(19) Europäisches Patentamt
European Patent Office
Office européen des brevets

(11) EP 0 736 009 B1

(12) EUROPEAN PATENT SPECIFICATION

(45) Date of publication and mention of the grant of the patent:
**11.09.2002 Bulletin 2002/37**

(21) Application number: **95906100.3**

(22) Date of filing: **22.12.1994**

(51) Int Cl.[7]: **C07D 211/94**, C07D 295/22, A61K 31/445

(86) International application number:
**PCT/US94/14850**

(87) International publication number:
**WO 95/017385 (29.06.1995 Gazette 1995/27)**

# (54) N-OXIDES OF 4-ARYLPIPERAZINES AND 4-ARYLPIPERIDINES AS ANTIPSYCHOTIC DRUGS

N-OXIDE VON 4-ARYLPIPERAZINEN UND 4-ARYLPIPERIDINEN ALS ANTIPSYCHOTISCHE MEDIKAMENTE

N-OXYDES DE 4-ARYLEPIPERAZINES ET 4-ARYLPIPERIDINES UTILISES COMME MEDICAMENTS NEUROLEPTIQUES

(84) Designated Contracting States:
**AT BE CH DE DK ES FR GB GR IE IT LI LU MC NL PT SE**
Designated Extension States:
**SI**

(30) Priority: **23.12.1993 US 173382**

(43) Date of publication of application:
**09.10.1996 Bulletin 1996/41**

(73) Proprietor: **ORTHO PHARMACEUTICAL CORPORATION**
**Raritan, NJ 08869 (US)**

(72) Inventors:
- **REITZ, Allen, B.**
  **Lansdale, PA 19446 (US)**
- **McDONNELL, Mark, E.**
  **Lansdale, PA 19446 (US)**

(74) Representative: **Mercer, Christopher Paul et al**
**Carpmaels & Ransford**
**43, Bloomsbury Square**
**London WC1A 2RA (GB)**

(56) References cited:
**WO-A-93/04682** **FR-A- 2 697 251**

- **XENOBIOTICA, vol.23, no.5, 1993 pages 495 - 508 GORROD, FANG 'On the metabolism of haloperidol'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Antipsychotic drugs are known to alleviate the symptoms of mental illnesses such as schizophrenia. Examples of such drugs include phenothiazine derivatives such as promazine, chlorpromazine, fluphenazine, thioridazine and promethazine, thioxanthenes such as chlorprothixene, butyrophenones such as haloperidol and clozapine. While these agents may be effective in treating schizophrenia, virtually all except clozapine produce extrapyramidal side effects, such as facial tics or tardive dyskinesia. Since antipsychotics may be administered for years or decades to a patient, such pronounced side effects may complicate recovery and further isolate the individual from society.

Compounds having some structural similarity to those of the present invention are described in EP-A-0296716 U. S. Patent Nos. 4,772,604; 4,782,061; 4,362,738; 3,988,371; 4,666,924; 4,931,443; and 4,992,441. Other somewhat similar compounds are disclosed in *J. Clin. Chem. Clin. Biochem.* **1988**, *26,* 105 and *J. Med. Chem.*, **1991,** *34,* 2133.

The present invention describes novel compounds that display activity in mammals suggestive of antipsychotic activity in man. The receptor binding profile shows only weak affinity for the dopamine-2 receptor, which may be an indication that the N-oxide functionality is being metabolically reduced *in vivo* to the corresponding piperazines and piperidines. Such piperazines and piperdines are disclosed and claimed in WO-A-9304682.

Compounds of the general formula **I**:

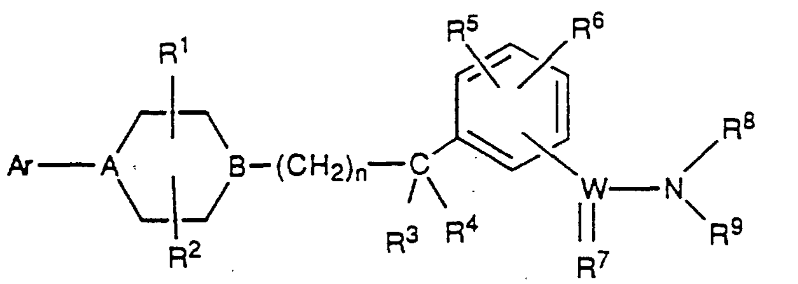

I

wherein Ar, W, A, B, $R^1$, $R^2$, $R^3$, $R^4$, $R^5$, $R^6$, $R^7$, $R^8$, $R^9$ and n, as defined hereinafter, are potent antipsychotic agents useful in the treatment of psychotic conditions such as schizophrenia in animals and humans. The compounds of the present invention may also be useful in the treatment of other disorders of the central nervous system such as anxiety and aggression.

Accordingly, the present invention provides compounds represented by the general formula I:

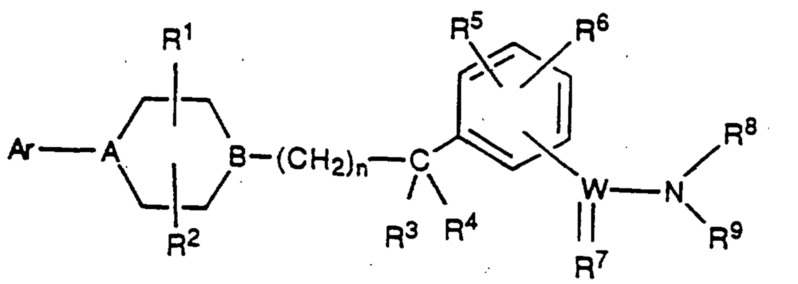

I

wherein Ar, W, A, B, $R^1$, $R^2$, $R^3$, $R^4$, $R^5$, $R^6$, $R^7$, $R^8$, $R^9$ and n are as defined in Claim 1.

Preferably, W is C.

Preferably, $R^1$ and $R^2$ are each H.

Preferably, n = 0.

Preferably, $R^3$ and $R^4$ are each H.

Preferably, $R^7$ is 0.

Where $-NR^8R^9$ is taken together to form a ring having 4-10 ring atoms, preferably the ring has 4-8 ring atoms, which ring is preferably saturated. Preferably, the additional hetero atoms in the ring are N or O; more preferably, the additional hetero atom is O; and most preferably, there are no additional hetero atoms. Where the $-NR^8R^9$ ring is combined with a four membered moiety to form a spirocycle ring system, this is preferably saturated. Preferably, the 2-4 membered carbon moiety is combined with a $-NR^8R^9$ ring which contains 5-7 ring atoms with the N being the only hetero atom in the ring, thereby forming a fused ring system. Preferably, the $-NR^8R^9$ ring is saturated prior to being

fused with the 2-4 membered carbon moiety. Preferably, $R^8$ and $R^9$ are taken together with the N to form a 6 membered fully saturated ring which may be substituted or unsubstituted.

The preferred substituents for the $-NR^8R^9$ ring are $C_1$-$C_8$ alkyl, hydroxy or oxo. The preferred substituents for the fused ring system are $C_1$-$C_4$ alkoxy. The spirocycle ring system is preferably unsubstituted and saturated.

Examples of preferred ring systems wherein $-NR^8R^9$ are taken together to form a ring having 4-10 ring atoms include pyrrolidine, piperidine, hexahydroazepine, octahydroazocine, oxazine and 2,6-dimethylpiperidine.

Examples of preferred fused ring systems for $-NR^8R^9$ are represented by formulas **III** and **IV**:

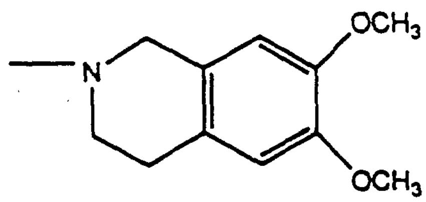

III

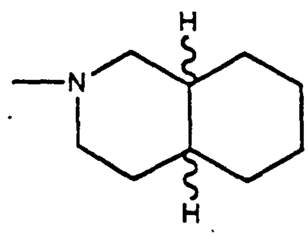

IV

As used herein for the definition of $-NR^8R^9$, a spiro ring system is a 2 ring system, the union of which is formed by a single atom which is the only common member of the two rings. A particularly preferred spirocycle ring is represented by the formula V:

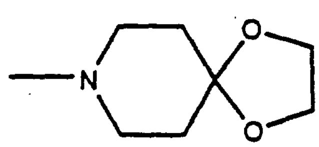

V

Alkoxy, such as i-propoxy or methoxy are presently the preferred substituents when Ar is substituted naphthyl or phenyl. As a halogen, the substitution is preferably fluorine, chlorine, or bromine. Optionally, present hydroxyl or hydroxyalkyl groups may be esterified or etherified. More preferably, Ar is substituted phenyl.

Ar may also be a fused ring system of the formula **II**:

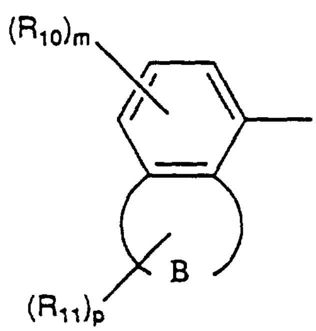

II

wherein B together with $R_{10}$, $R_{11}$, m and p are as defined below.

More preferred values for the moiety of formula II are: B forms together with the two carbon atoms of the phenyl group an entirely or partly unsaturated ring consisting of 5 atoms, which ring comprises at least one oxygen atom. $R^{10}$ and $R^{11}$ are selected from any of $C_1$-$C_5$ alkyl, $C_1$-$C_5$ alkoxy, hydroxyl, nitro, cyano, halogen, or trifluoromethyl. $R^{10}$ and $R^{11}$ are more preferably selected from any of $C_1$-$C_5$ alkoxy, halogen or cyano. $R^{10}$ is preferably in the meta or ortho position in relation to the piperazine/piperidine group. Variables m and p have the value 0-2. A particular preferred subgensis of such compounds are those wherein m and p each have a value of 0.

When $R^{10}$ or $R^{11}$ comprises an alkyl group, it is a straight or branched alkyl group having 1-5 carbon atoms. As a cycloalkyl group, the groups $R^{10}$ or $R^{11}$ comprise a ring system having 3-7 ring atoms and not more than 10 carbon atoms including any substituents as a whole. When $R^{10}$ or $R^{11}$ is a hydroxyalkyl group such a group comprises 1-5 carbon atoms. As a halogen atom, $R^{10}$ or $R^{11}$ preferably is fluorine, chlorine or bromine. Optionally present hydroxyl or hydroxyalkyl groups may be esterified or etherified.

When $R^{10}$ or $R^{11}$ is substituted phenyl it may be substituted with one or more of $C_1$-$C_8$ alkyl, $C_1$-$C_8$ alkoxy, halogen, trifluoromethyl, $C_1$-$C_8$ alkylthio, dialkylamino (wherein each alkyl is $C_1$-$C_8$), $C_1$-$C_8$ alkylamino, nitro or mono- or dialkylamino sulfonyl (wherein each alkyl is $C_1$-$C_8$).

As used herein, unless otherwise noted alkyl and alkoxy whether used alone or part of a substituent group, include straight and branched chains. For example, alkyl radicals include methyl, ethyl, propyl, isopropyl, n-butyl, isobutyl, sec-butyl, t-butyl, n-pentyl, 2-methyl-3-butyl, 1-methylbutyl, 2-methylbutyl, neopentyl, n-hexyl, 1-methylpentyl, 2-methylpentyl. Alkoxy radicals are oxygen ethers formed from the previously described straight or branched chain alkyl groups. Of course, if the alkyl or alkoxy substituent is branched there must be at least 3 carbon atoms.

The term "aryl" as used herein alone or in combination with other terms indicates the aromatic hydrocarbon groups phenyl or naphthyl. The term "heteroaryl" means aromatic hydrocarbon groups containing 1 or 2 hetero atoms selected from any of S, O or N. The term "aralkyl" means a $C_1$-$C_8$ alkyl group substituted with an aryl group. The term acyl, unless otherwise specified herein, means a benzoyl or a $C_1$-$C_8$ alkanoyl group, which can be optionally substituted. With reference to subsliuents, the term independently means that when more than one of such substituent is possible such substituents may be the same or different from each other.

Compounds according to this invention have a 1,2-, 1,3- or 1,4-relationship of the W substituent with the -C($R^3$)($R^4$)- group on the W-bearing phenyl ring. Preferred compounds have a 1,2- or 1,3- relationship of these two groups. The $R^5$ and $R^6$ substituents may be located in any of the other unsubstituted ring positions.

Particularly preferred subgenuses of compounds of the formula I are those of the formula (**Ia-Ic**):

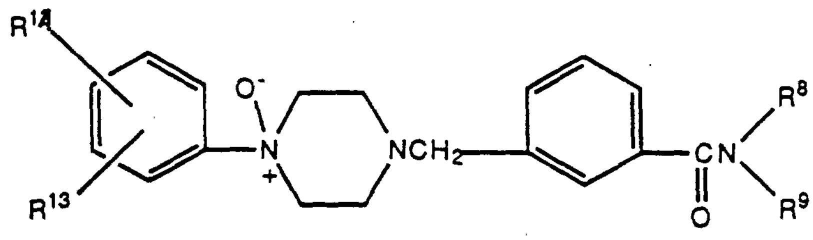

Ia

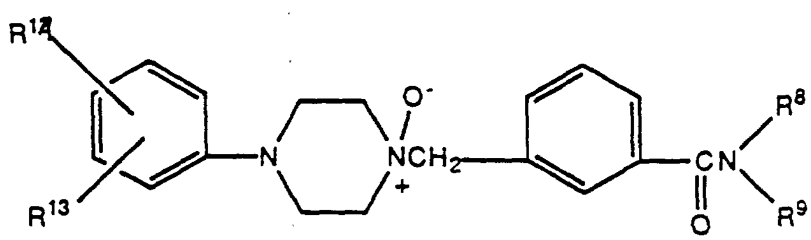

Ib

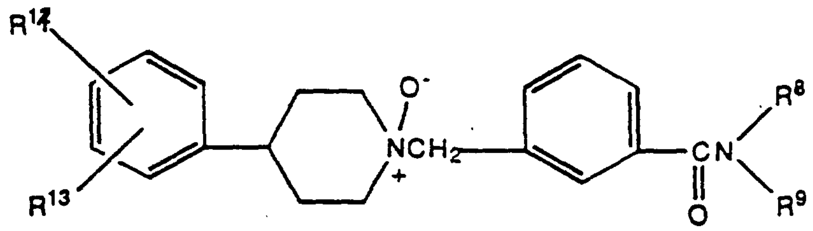

Ic

wherein $R^8$ and $R^9$ are as defined above and $R^{14}$ and $R^{13}$ are as defined as substituents for Ar in formula **I**. Preferably, $R^8$ and $R^9$ are taken together with the N to form a saturated ring having 5-8 ring atoms and one of $R^{14}$ and $R^{13}$ is $C_1$-$C_8$ alkoxy and the other is H. The most preferred $C_1$-$C_8$ alkoxy groups are i-propoxy or methoxy.

Examples of particularly preferred compounds include:

1-[3-[[4-[2-(1-Methylethoxy)phenyl]-4-oxide-1-piperazinyl]methyl]-benzoyl]piperidine;

1-[3-[[4-[2-(1-Methylethoxy)phenyl]-1-piperazinyl-1-oxide]methyl]-benzoyl]piperidine; and

1-[3-[[4-[2-(1-Methylethoxy)phenyl]-1-piperidinyl-1-oxide]methyl]-benzoyl]piperidine monohydrochloride.

The invention definition of formula **I** includes racemates and individual optical isomers, e.g. as caused by the presence of a stereogenic carbon such as when a substituent would be 2-butyl. Also within the scope of the invention are compounds of the invention in the form of hydrates and other solvate forms.

Representative salts of the compounds of formula **I** which may be used include those made with acids such as hydrochloric, hydrobromic, hydroiodic, perchloric, sulfuric, nitric, phosphoric, acetic, propionic, glycolic, lactic, pyruvic, malonic, succinic, maleic, fumaric, malic, tartaric, citric, benzoic, cinnamic, mandelic, methanesulfonic, ethanesulfonic, hydroxyethanesulfonic, benzenesulfonic, p-toluenesulfonic, cyclohexanesulfamic, salicyclic, p-aminosalicyclic, 2-phenoxybenzoic, 2-acetoxybenzoic or a salt made with saccharin. Such salts can be made by reacting the free base of formula **I** with the acid and recovering the salt.

The compounds of formula **I** may be prepared according to Reaction Scheme 1.

## Reaction Scheme 1

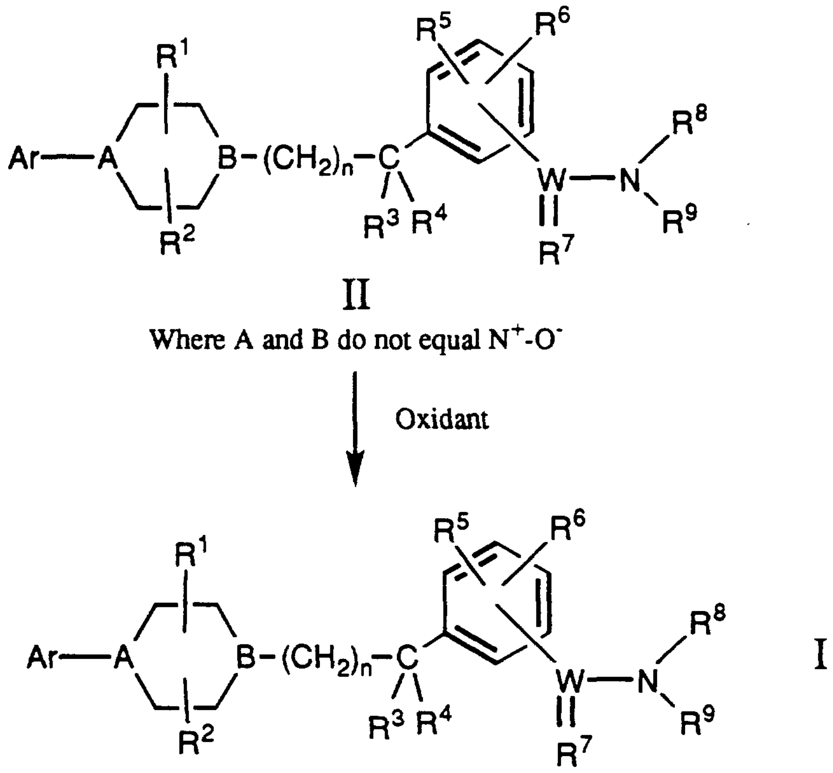


II

Where A and B do not equal $N^+$-$O^-$

I

Where A and B are as defined in herein

As shown, oxidant refers to any reagent or reagent mixture capable of transfering an oxygen to a nitrogen atom to convert it to the N-oxide functionality. Examples of such reagents are peracids, such as m-choroperoxybenzoic acid and peracetic acid. Suitable solvents would include halogenated solvents such as methylene chloride and chloroform. Another reagent capable of effecting this transformation is ruthenium on carbon (5 %) in the presence of sodium acetate, acetic acid, and peracetic acid. Occasionally, the products can be obtained immediately from the reaction without the need for chromatographic purification. However, when there are two possible sites of oxidation (e.g. with a piperazine, A = B = N), the two different singly oxidized compounds (A = $N^+$-$O^-$, B = N and B = N, A = $N^+$-$O^-$) can be separated from each other by chromatographic methods such as by flash column chromatography on silica gel.

The requisite piperazines and piperidines of formula **II** described herein and in Examples 1-3 are prepared as described in WO 9304682 (18 Mar 1993), being a CIP of WO 9304684 (18 Mar 1993). More specifically, the requisite piperazines and piperidines of formula **II** may be prepared as described in Reaction Schemes 2-8.

The compounds of formula II may be prepared according to Reaction Scheme 2:

## Reaction Scheme 2

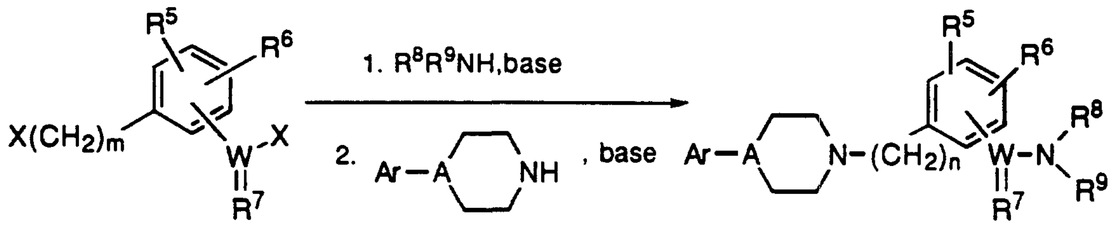


X = Cl, Br

VI

VII

VIII

As shown, the 1,2-, 1,3-, and 1,4-disubstituted benzamides or sulfonamides may be prepared by a sequential reaction with the appropriate haloalkyl benzoyl halide or haloalkyl benzenesulfonyl halide (**VI**). The first condensation with the requisite amine is conducted in a non-protic solvent such as tetrahydrofuran (THF) with cooling (e.g. in the range -78°C to 5°C), being careful not to let the solution exotherm so as to avoid reaction of the haloalkyi functionality. The base present in the reaction (for the removal of the HX formed) is typically a tertiary amine such as triethylamine or diisopropylethylamine, or it could be a molar excess (at least) of the amine reactant (e.g. $R^8R^9NH$). The intermediate haloalkyl benzamide thus formed could be then taken on directly to the product by reaction with the aryl piperazine or aryl piperidine (**VII**), or it could be isolated after an extractive workup and/or chromatography. If the intermediate was carried on in situ to the product (**VIII**) in THF, heating (30°C-67°C) is generally required for complete reaction. If the intermediate is isolated and then reacted separately with the aryl piperazine or aryl piperidine, the optimal solvents are dipolar aprotic solvents such as dimethylformamide (DMF) or N-methyl-2-pyrrolidinone. The base used in this latter step could be a tertiary amine or potassium or sodium carbonate. Using the two-step method (i.e. isolation of the intermediate), the product could in some cases be obtained pure after recrystallization as a salt without resort to chromatography.

1,2- and 1,3-Halomethylbenzoyl halides used when m=1 in Reaction Scheme 2 are commercially available from Fluka, Carbolabs or Pfaltz and Bauer, or could be prepared by literature methods or modifications thereof. (See e.g.: Ger. Often. 2,835,440, 28 Feb. 1980; and J. Johnson and I. Pattison *J. Hetero. Chem.* **1986,** *23,* 249). Halomethyl benzoyl halides bearing substituents have also been described in the literature, such as in the methoxy-substituted case cited in R. Quelet et al. *Bull. Soc. Chem., France* **1969,** 1698. The final products are typically chromatographed to achieve purity, and then converted to an acceptable salt form.

The 1,3- or 1,4-disubstituted analogs may be prepared in the same manner as the derivatives shown above. There are alternative methods for the preparation of compounds of this type. For example, they may be synthesized by a palladium-mediated coupling of a bromoaryl derivative with carbon monoxide and piperidine (*J. Org. Chem.* **1974,** *39*, 3327) as shown in Reaction Scheme 3 for a 1,4-disubstituted case.

## Reaction Scheme 3

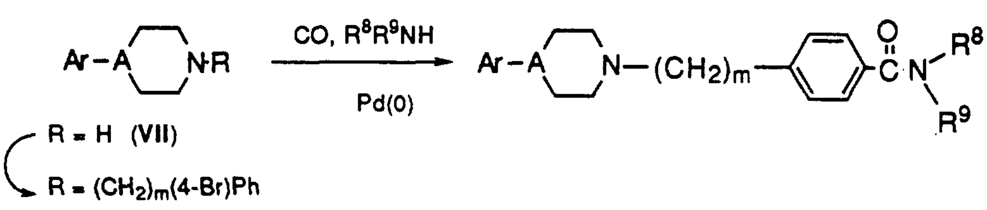


IX

X

The preparation of the sulfonamide analogues (W = SO, $R^7$ = O, and n = 0 in **II**) require preparation of the necessary halomethyl sulfonyl halide by halogenation of the appropriate toluenesulfonyl halides on the benzylic methyl position with N-bromosuccinimide mediated by benzoyl peroxide. The halomethyl sulfonyl halides were used in generally the same manner as for the benzoyl halide case (e.g. see Reaction Scheme 3).

Many aryl piperazines are commercially available from Aldrich Chemical Company or may be prepared by standard methods known in the art (for example see G. E. Martin et al. *J. Med. Chem.* **1989,** *32*, 1052). These piperazines (VII, A=N) may be obtained according to the following Reaction Scheme 4 where Ar is as described in connection with formula **II** and Z is a leaving group such as halo (e.g. chloro):

## Reaction Scheme 4

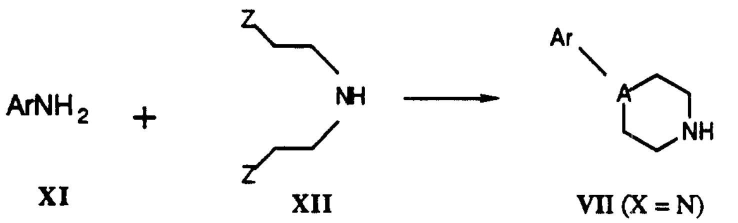

XI XII VII (X = N)

In carrying out Reaction Scheme 4, compound **XII** is heated with an aniline or an aromatic heterocyclic primary amine **XI** at about 50 to 120°C in a solvent such as n-butanol with recovery of the piperazine **VII** (A=N).

Piperazines of formula **VII** (A=N) where Ar is a formula **II** moiety are described as formula (2) in U.S. Patent 4,782,061 published earlier as EPO 185,429 and EPO 190,472 on June 15, 1986 and August 13, 1986, respectively, which documents are hereby incorporated by reference. Other piperazines of formula **VII** (A=N) where Ar is a formula **II** moiety are described as formula 29 in EPO 138,280 published April 24, 1985 which is incorporated by reference. In addition, some of the piperazines of formula **VII** can be prepared by the method of ten Hoeve et al. (*J. Org. Chem.* **1993,** 58, 5101), involving the displacement of a methoxy aromatic by piperazine or a piperazine derivative.

Other piperazines may be prepared by the method of I. van Wijngaarden et al. (*J. Med. Chem.* **1988,** *31,* 1934). Other piperidines may be prepared by the method shown in Reaction Scheme 5.

## Reaction Scheme 5

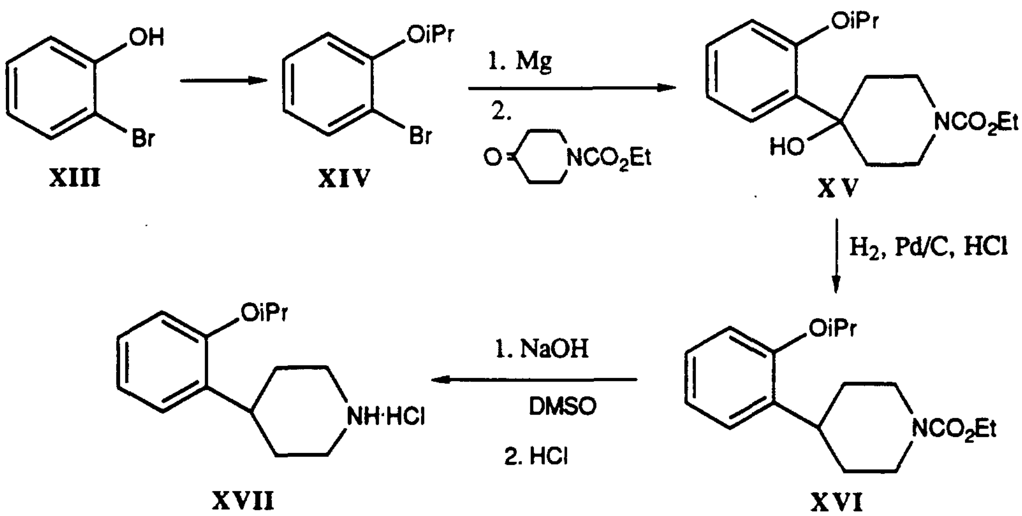


XIII XIV XV XVI XVII

Other piperazine may be synthesized as shown in Reaction Scheme 6.

## Reaction Scheme 6

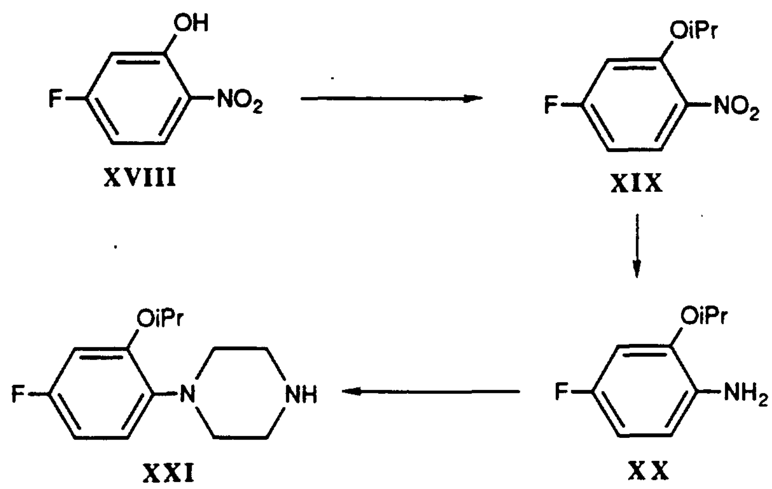

The piperazines required to prepare 2-fluoropiperazinyl compounds may be prepared by nucleophilic displacement of 1,2-difluorobenzene with the requisite piperazine such as in reaction of 2,5-dimethylpiperazine with 1,2-difluorobenzene in the presence of sodium amide.

Alternatively, certain other compounds useful in making the compounds of the invention can be prepared by the method shown in Reaction Scheme 7.

## Reaction Scheme 7

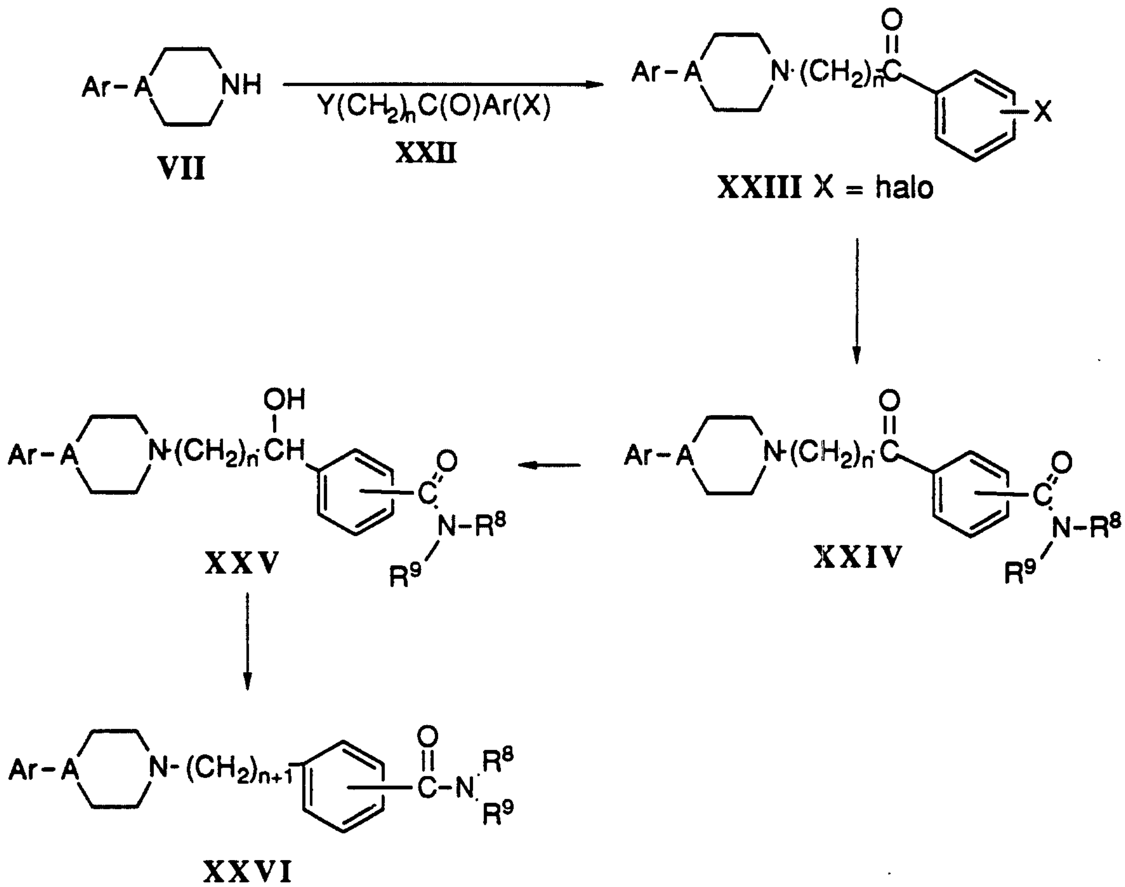


VII

XXII

XXIII X = halo

XXV

XXIV

XXVI

Aryl piperazines **VII** (A=N) can be condensed with compounds **XXII** in which Y represents a leaving group suitable for a diplacement reaction (e.g. halogen, p-toluenesulfonate, trifluoromethanesulfonate) to give compounds **XXIII**. This deplacement reaction is typically carried out in a dipolar aprotic solvent such as DMSO or DMF, using sodium carbonate, potassium carbonate, or a tertiary amine [e.g. triethylamine or di(isopropyl)ethylamine] as the base, generally with heating (30-80°C for 2h to 4d). The resulting ketone (**XXIII**) can be converted to amide **XXIV** by the arninocarbonylation reaction described for Reaction Scheme 3. Reduction of the carbonyl group of **XXIV** by the use of sodium borohydride in alcoholic solvents (EtOH, iPrOH) at room temperature (2-30h) can gave alcohol **XXV**. Further reduction of **XXV** by the method of catalytic hydrogenation (H2, palladium/carbon) in alcoholic solvents (e.g. EtOH), in the presence of added mineral acid (e.g. HCl) to facilitate the reaction, can afford compounds **XXVI**.

Compounds of formula II may also be prepared by the chemistry shown in Reaction Scheme 8.

## REACTION SCHEME 8

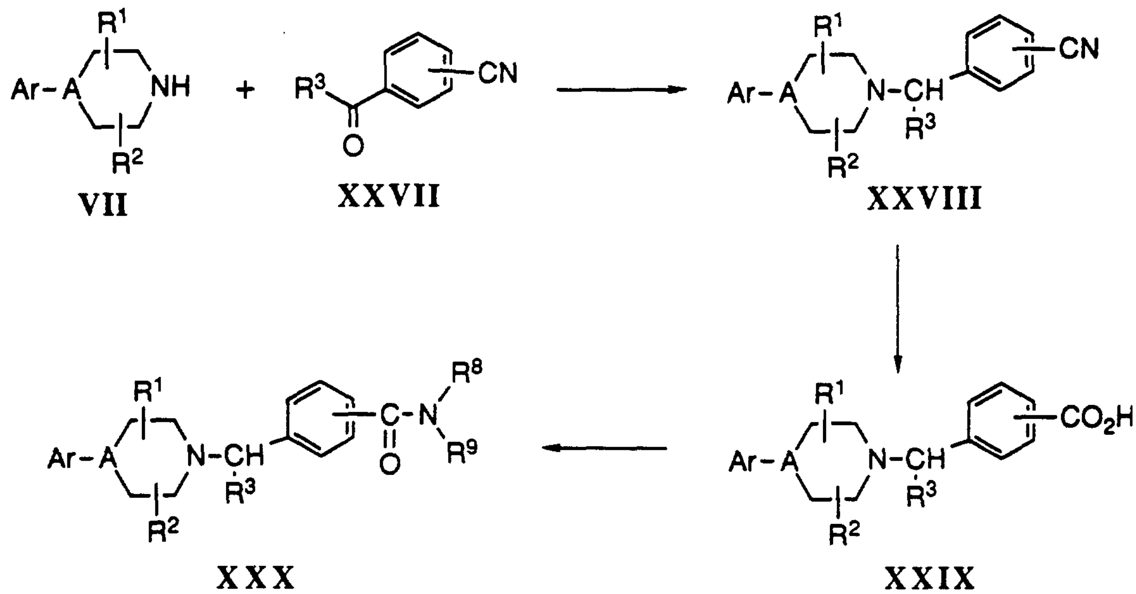

VII XXVII XXVIII

XXX XXIX

Carbonyl compound **XXVII** is reacted with compounds **VII** in a reductive amination reaction to give compounds **XXVIII**. This reaction can be carried out using sodium borohydride in titanium isopropoxide. It can also be conducted by forming an imine from **VII** and **XXVII** and then reducing it catalytically with hydrogen in the presence of a noble metal catalyst (e.g. palladium or platinum). Hydrolysis of the nitrile functionality of **XXVIII** to give **XXIX** is carried out in the presence of sodium hydroxide or potassium hydroxide, usually at reflux in an alcoholic solvent. Compound **XXIX** is then combined with $R^8R^9NH$ to form amide **XXX,** using one of the standard reactions to accomplish this transformation such as the use of dicyclohexylcarbodiimide or carbonyl diimidazole.

The antipsychotic activity of the compounds of the invention may be determined by the Block of Conditioned Avoidance Responding (Rat) test (CAR), references being Cook, L. and E. Weidley in *Ann. N.Y. Acad. Sci.,* **1957,** 6, 740-752, and Davidson, A.B. and E. Weidley in *Life Sci.,* **1976,** 18, 1279-1284. This test was performed for compounds disclosed in this invention, and the data are listed in Table 1. A reading of -20% in the CAR test was generally taken to represent a minimum value for a compound to be designated as active at a given dose. In addition, the affinity of the compounds for several receptors found in the central nervous system was evaluated; the affinity for the D-2 (dopamine-2) receptors is also listed in Table 1. As modulation of this receptor is generally recognized to be beneficial in the treatment of schizophrenia (G. P. Reynolds *Trends Pharmacol. Sci.* **1992,** *13,* 116), affinity for this receptor indicates potential utility for the compounds. A D-2 affinity of 1000 nM or less has been taken as predictive of antipsychotic activity.

Block of Conditioned Avoidance Responding (Rat)

Apparatus: Rat operant chambers, housed within sound attenuated booths, both from Capden Instruments Ltd., were used in this test. The test chamber (8" H x 90-3/8" W x 9" D) is constructed of aluminum and plexiglass with floor grid bars of stainless-steel (1/8" O.D.) spaced 9/16" apart. A stainless-steel operation level 1-1/2" wide projects 3/4" into the chamber and is positioned 2-2/8" above the grid floor. The shock stimulus is delivered via the grid floor by a Coulbourn Instruments solid state module. The parameters of the test and the collection of data are controlled automatically.

Training: Male, Fischer 344 rats obtained from Charles River (Kingston, NY) weighing more than 200 g, are individually housed with chow and water provided ad libitum. The rats are trained for two weeks to approach criterion levels in the avoidance test (90% avoidance rate). One-hour training sessions are run at about the same time each day for four or five days a week. The training session consists of 120 trials, with the conditioned stimuli presented every 30 sec. A trial begins with presentation of the conditioned stimuli (a light and a tone). If the rat responds by depressing the operant lever during the 15-second presentation of the conditioned stimuli, the trial is terminated and the animal is credited with a CAR. Failure to respond during the conditioned stimuli causes the presentation of the unconditioned

stimulus (UCS), a 0.7 mA shock which is accompanied by a light and tone for five seconds. If the rat depressed the lever within the ten-second period, the shock and trial are terminated and an escape response recorded. If the rat fails to depress the lever during the UCS (shock), the trial is terminated after ten seconds of shock and the absence of a response is scored as a failure to escape. Intertrial level presses have no effect. If a rat performs at the 90% CAR level for two weeks, it is then run twice a week on the test schedule (see below) until baseline performance stabilized. Before any drug is administered, two weeks of CAR at a rate of 90% or better is required.

The percent change in CAR on the drug treatment day compared to vehicle pretreatment day is the key measure. The percent change (% change) in CAR is determined using the following formula:

$$\% \text{ change CAR} = ((\% \text{ CAR for Day 2}/\% \text{ CAR for Day 1}) \times 100)-100$$

A negative number indicates a blockade of CAR, whereas a positive number would indicate increased CAR. The test results are reported as the mean % change for the group of rats. Failure to escape, a measure of the general sedative potential of the compound, was calculated for each animal as follows:

$$\% \text{ Failures} = \#\text{ of Failures to Escape}/\#\text{ of trials}$$

The % failures, viz., loss of escape, is also reported as a group mean. Failures to escape are monitored closely and a session is terminated if ten failures occurred. $ED_{50}$ values and 95% confidence limits are calculated using linear regression analysis. The results of the CAR tests are shown in Table I.

In the Table and formulas therein, OiPr is isopropoxy, Me is methyl, and NT is not tested in that particular test. The escape loss numbers are shown at CAR 15 mg/kg ip.

Receptor Binding Assay

The dopamine $D_2$ binding activity of compounds was determined using a $P_2$ fraction (synaptosomal membranes) prepared from male, Wistar rats. The $D_2$ assay employed a $P_2$ fraction from the striatum, the ligand $^3$H-spiperone at a concentration of 0.05 nM, and 1 mM haloperidol as a blank determinant. Incubation was in 3 mM potassium phosphate buffer for 45 min at 37°C. Under these conditions, specific binding constituted 75% of total binding, and the $K_I$ values for some known drugs were: 0.37 nM for haloperidol and 82 nM for clozapine.

The data from this assay were analyzed by calculating the percent inhibition of the binding of the tritiated ligands by given concentrations of the test compound. $K_I$ values, where given, were obtained from the logit analysis of concentration-inhibition curves.

To prepare the pharmaceutical compositions of this invention, one or more compounds or salts thereof of the invention, as the active ingredient, is intimately admixed with a pharmaceutical carrier according to conventional pharmaceutical compounding techniques, which carrier may take a wide variety of forms depending on the form of preparation desired for administration, e.g., oral or parenteral. In preparing the compositions in oral dosage form, any of the usual pharmaceutical media may be employed. Thus for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules and tablets, suitable carriers and additives include starches, sugars, diluents, granulating agents, lubricants, binders, disintegrating agents and the like. Because of their ease in administration, tablets and capsules represent the most advantageous oral dosage form, in which case solid pharmaceutical carriers are obviously employed. If desired, tablets may be sugar coated or enteric coated by standard techniques. For parenterals, the carrier will usually comprise sterile water, though other ingredients, for example, for purposes such as aiding solubility or for preservation, may be included. Injectable suspensions may also be prepared, in which case appropriate liquid carriers, suspending agents and the like may be employed. The pharmaceutical compositions herein will preferably contain per dosage unit, e.g., tablet, capsule, powder, injection, teaspoonful and the like, from about 50 to about 100 mg of the active ingredient, although other unit dosages may be employed.

In therapeutic use as an antipsychotic agent in mammals, the compounds of this invention may be administered in an amount of from about 0.5 to 5 mg/kg per day, and more preferably 1-3 mg/kg per day. The dosages, however may be varied depending upon the requirements of the patient, the severity of the condition being treated and the compound being employed. Determination of optimum dosages for a particular situation is within the skill of the art.

The following Examples illustrate the present invention, but are not deemed to be limiting. Examples 1-3 describe the preparation of specific compounds listed in the Table which follow the Examples.

**EXAMPLE 1**

1-[3-[[4-[2-(1-Methylethoxy)ehenyl]-4-oxide-1-piperazinyl]methyl]benzoyl]piperidine 1,7Hydrate (Compound 1)

1 -[3-[[4-[2-(1-Methylethoxy)phenyl]-1-piperazinyl]methyl]benzoyl]-piperidine (15 g, 35.6 mmol) was treated with m-chloroperoxybenzoic acid (6.15 g, 35.6 mmol) dissolved in chloroform (100 mL). This solution was allowed to stir overnight, and the solvent was then removed. The residue was purified on a silica gel column ($CHCl_3$/MeOH; 100: 0 to 90:10). The first component isolated consisted of unreacted starting material (9.8 g). The second component isolated (380 mg) was recrystallized from methylene chloride/ether, and was identified to be the title compound by analysis of the H-1 NMR spectrum (400-MHz, $CD_3OD$) and mass spectrum as a white powder, mp 98-101°C. The compound was labile to prolonged heating in methanol, and was kept in the refridgerator. $^1$H NMR ($CD_3OD$, 250 MHz) δ 8.4 (d, 1H), 7.4 (m, 4H), 7.3 and 7.2 (both d, 1H each), 7.1 (t, 1H), 4.9 (m, 3H), 3.7 (d, 4H), 3.4 (s, 2H), 3.15 (t, 2H), 2.8 and 2.9 (both d, 2H each), 1.7 (m, 4H), 1.5 (d, 6H).

Elemental Analysis: Calculated for $C_{26}H_{35}N_3O_3 \cdot 1.7H_2O$: C, 66.71; H, 8.21; N, 8.98; $H_2O$ 6.54. Found: C, 66.90; H, 7.97; N, 8.62; $H_2O$, 6.15.

**EXAMPLE 2**

1-[3-[[4-[2-(1-Methylethoxy)phenyl]-1-piperazinyl-1-oxide]methyl]benzoyl]piperidine 1.1 Perchlorate 0.4Hydrate (Compound **2**)

The third component to be isolated from the chromatography described in Example 1 was the title compound listed, 2.62 g, indicating a 3.4:1 preference for oxidation of the benzylic amine relative to the aniline nitrogen. This material was dissolved in MeOH (ca. 10 mL) and treated with 70% aqueous perchloric acid, and then triturated with ether, causing the perchlorate salt of the title compound to emerge. Analysis of the H-1 NMR spectrum (400-MHz, $CD_3OD$) and mass spectrum of the compound confirmed the structure indicated. $^1$H NMR ($CD_3OD$, 250 MHz) δ 7.65 (d, 1H), 7.6 (m, 3H), 7.0 (m, 3H), 6.9 (t, 1H), 4.95 (s, 2H), 4.65 (q, 1H), 4.0 (t, 2H), 3.7 (m, 4H), 3.6 (d, 2H), 3.35 (m, 4H), 1.7 (m, 4H), 1.55 (m, 1 H), 1.35 (d, 6H).

Elemental Analysis: Calculated for $C_{26}H_{35}N_3O_3 \cdot 1.1\ HClO_4 \cdot 0.4H_2O$: C, 56.23; H, 6.65; N, 7.56; Cl, 7.02; $H_2O$, 1.29. Found: C, 56.01; H, 6.64; N, 7.08; Cl, 7.36; $H_2O$, 1.21.

**EXAMPLE 3**

1-[3-[[4-[2-(1-Methylethoxy)phenyl]-1-piperidinyl-1-oxide]methyl]benzoyl]-cis-2.6-dimethylpiperidine 0.6Hydrate 0.25Chloroform (Compound **3**)

A mixture of 2-bromophenol (23.2 mL, 0.20 mol), potassium carbonate (33.2 g, 0.24 mol) and 2-bromopropane (28.0 mL, 0.30 mol) in dimethylformamide (200 mL) was stirred in a preheated oil bath (60°C) for 5 h. The cooled reaction mixture was then partitioned between ether and water. The layers were separated and the aqueous phase was extracted with ether. The combined organic solution was washed with copious amounts of water, 3N aqueous NaOH, dried ($MgSO_4$), filtered and concentrated in vacuo to furnish 39.3 g (91%) of 2-(isopropoxy)bromobenzene as a pale yellow oil which was carried on without further purification. The structure was supported by GC/MS and 90 MHz $^1$H NMR.

To a suspended solution of Mg chips (10.07 g, 0.414 mol) in anhydrous ether (150 mL) at 22°C under argon was added ca. 0.15 mL of 1,2-dibromoethane. Then 43.7 g (0.200 mol) of 2-(isopropoxy)bromobenzene in 200 mL of ether was added dropwise. After 50% of the aryl halide was added, the reaction began to reflux vigorously. The flask was cooled in an ice bath. After the refluxing had subsided somewhat, the ice bath was removed and the remaining aryl halide was added over a 1.5 h period. The resultant Grignard reagent was cooled in a dry ice/ether bath for 2 h and then treated with 34.0 mL (0.221 mol) of 98% 1-carbethoxy-4-piperidone. Upon complete addition of ketone, the reaction mixture was allowed to warm to 22°C and stirred for 2 h. The reaction was then quenched with cold aqueous ammonium chloride which resulted in an emulsion. Addition of 1M aqueous HCl solution separated the two layers. The aqueous phase was extracted with additional ether and the combined organic solution was washed with 10% aqueous sodium bisulfite, 1.0 M HCl, saturated $NaHCO_3$, and dried ($K_2CO_3$). Filtration and concentration yielded 56.36 g of 1-carbethoxy-4-[2-(1-methylethoxy)phenyl]-4-piperidinol as a yellow viscous oil which was carried on without further purification The structure of this oil was supported by $^1$H NMR.

A crude solution of 1-carbethoxy-4-[2-(1-methylethoxy)phenyl]-4-piperidinol (36 g), 10% palladium on carbon (1.80 g), 5 mL of concentrated HCl and 125 mL of MeOH was shaken on a Parr apparatus under 55.5 psig of hydrogen at 22°C for 3 d. The reaction was filtered over Celite, and concentrated to a residue. This material was partitioned

between ether and water. The organic solution was dried ($MgSO_4$), filtered, and concentrated to yield 29.34 g of 1-carbethoxy-4-[2-(1-methylethoxy)phenyl]piperidine as a light yellow oil which was carried forward without further purification. The structure was supported by MS and $^1H$ NMR.

A mixture of crude 1-carbethoxy-4-[2-(1-methylethoxy)phenyl]piperidine (29.3 g) and sodium hydroxide pellets (6.12 g, 0.106 mol) in DMSO (100 mL) was stirred in a preheated oil bath at 100°C for 4 d. The reaction mixture was then poured into water (200 mL) and the crude product was extracted into methylene chloride. The methylene chloride extracts were dried over $MgSO_4$, filtered and concentrated to afford 21.34 g of a crude dark brown oil. This oil was dissolved in 1N aqueous HCl solution and washed with ether. The acidic aqueous solution was basified with 3N NaOH and the product was extracted into methylene chloride. The combined methylene chloride extracts were dried ($MgSO_4$), filtered and concentrated to yield 13.34 g of a semi-solid. This material was dissolved in iPrOH and acidified to a pH of 3 with concentrated HCl. The acidified solution was diluted with ether resulting in precipitation of the monohydrochloride salt which was collected by filtration and dried under vacuum to provide 11.21 g of 4-[2-(1-methylethoxy)phenyl]piperidine hydrochloride as a beige powder. The structure was supported by MS. The free base was obtained by extraction into $CHCl_3$ from 1N NaOH, drying ($MgSO_4$), and filtration.

In 100 ml of 2-pyridone, 4-(2-isopropoxyphenyl)piperidine (5.69 g, 0.0259 mol), 3-(chloromethyl)benzoyl-2,6-cis-dimethylpiperidine (6.91 g, 0.0259 mol), and sodium carbonate (8.75 g, .0825 mol) were combined and heated to 70°C. After 2.5 hours, the oil bath was removed and the reaction cooled to room temperature while stirring overnight. The crude reaction mixture was diluted with chloroform and then subjected to flash chromatography on silica with chloroform as eluant resulting in the isolation of slightly impure product (7.95 g; 68%). Conversion to its corresponding. HCl salt, followed by neutralization provided pure free-base product. The assigned structure was supported by NMR and MS. Elemental Analysis calculated for $C_{29}H_{40}N_2O_2 \cdot 0.25H_2O \cdot HCl$: C, 71.14; H, 8.54; N, 5.72; Cl, 7.24; $H_2O$, 0.92. Found: C, 71.20; H, 8.83; N, 5.59; Cl, 6.99; $H_2O$, 0.55.

A solution of m-chloroperoxybenzoic acid (0.80 g, 4.63 mmol) in $CHCl_3$ (10 mL) was added dropwise to a suspended mixture of 1-[3-[[4-[2-(1-methylethoxy)phenyl]-1-piperidinyl-1-oxide]methyl]benzoyl]-cis-2,6-dimethylpiperidine in $CHCl_3$ (10 mL) while stirring at 0°C. The reaction was continued overnight. The solvent was then removed and the residue was purified on a silica gel column ($CHCl_3$/MeOH, 100:0 to 90:10). The solid which was obtained was recrystallized from $CHCl_3$/hexane to give the title compound as a white powder (0.70 g), m.p. 104-106°C. $^1H$ NMR ($CD_3OD$, 400 MHz) $\delta$ 7.7 (s, 1H), 7.58 (d, 1H), 7.53 (t, 1H), 7.45 and 7.35 (both d, 1H each), 7.14 (t, 1H), 6.95 (d, 1H), 6.9 (t, 1H), 4.6 (m, 1H), 4.45 (s, 2H), 3.5 (t, 2H), 3.2 (m, 2H), 2.4 (q, 2H), 1.93 (m, 1H), 1.7 (m, 10 H), and 1.3 (d, 6H). The mass spectra also supported the assigned structure.

Elemental Analysis calculated for $C_{29}H_{40}N_2O_3 \cdot H_2O \cdot CHCl_3$: C, 69.50; H, 8.20; N, 5.54; $H_2O$, 2.18. Found: C, 69.23; H, 8.36; N, 5.43; $H_2O$, 2.09.

## TABLE 1

| Structure | Compd. # | % CAR at 15 mg/kg (Loss of escape) | Dopamine-2 (nM) |
|---|---|---|---|
| 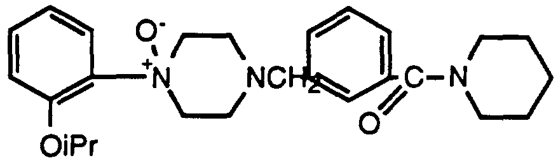 | 1 | -91.7 (44.8) | 140 |
| 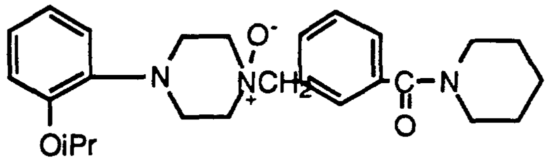 | 2 | -87.6 (31.5) | 355 |
| 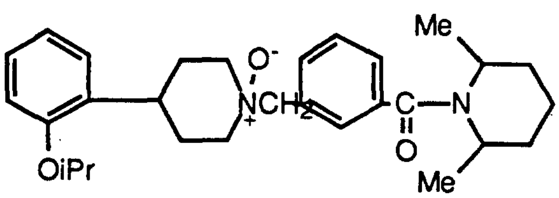 | 3 | -37.7 (0.4) | 371 |

## Claims

**1.** A compound represented by the formula I:

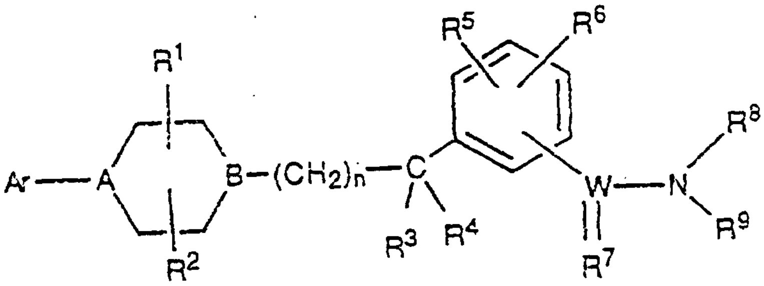

I

wherein

Ar is: phenyl or naphthyl, optionnaly substituted with one or more of $C_1$ - $C_8$ alkyl, cycloalkyl, $C_1$ - $C_8$ alkoxy, phenoxy, naphthoxy, hydroxyl, trifluoromethyl, trifluoromethoxy, cyano, $C_1$ - $C_8$ alkylthio, halogen, nitro, $C_1$ - $C_8$ haloalkyl amino or mono- or dialkylamino wherein each alkyl is $C_1$ - $C_8$; pyrimidinyl; pyridinyl; pyridazinyl; pyrazinyl; imidazyl; pyrrole; furan; thiophene; triazolyl; thiazolyl; or a fused ring system of the formula II:

II

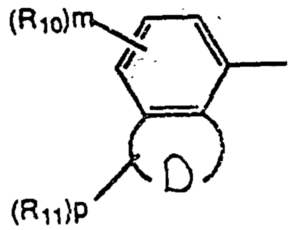

II

A is: N; CH; or $N^+$-$O^-$,
B is: N; or $N^+$-$O^-$;
with the proviso that when A is N or CH, B must be $N^+$-$O^-$ and when A is $N^+$-$O^-$, B must be N,
D together with the 2 carbon atoms of the phenyl group forms an entirely or partly unsaturated cyclic group having 5 - 7 ring atoms and within the ring 0 - 3 hetero atoms from any of O, S or N, with the proviso that the sum of the number of O and S atoms is at most 2, whereas the N atoms in the ring may be substituted with $R^{12}$,
W is C or SO,
$R^1$ and $R^2$ are: H; or $C_1$ - $C_4$ alkyl,
m = 0 - 3,
n = 0 - 4,
p = 0 - 2,
$R^3$ and $R^4$ are: either both H; or one of them is H and the other is $C_1$ - $C_4$ alkyl or hydroxyl; or both are taken together as oxygen to constitute a carbonyl group, with the proviso that when n = 0, $R^3$ and $R^4$ cannot be taken together as oxygen,
$R^5$ and $R^6$ are independently: H; $C_1$ - $C_8$ alkyl; $C_1$ - $C_8$ alkoxy; nitro; halogen; haloalkyl; $C_1$ - $C_8$ alkylthio; amino; $C_1$ - $C_8$ mono- or di- alkyl amino; or $C_1$ - $C_8$ alkylamido,
$R^7$ is: O or S where W is C; or O where W is SO,
$R^8$ and $R^9$ are independently: H; $C_1$ - $C_8$ alkyl; $C_1$ - $C_8$ aminoalkyl; phenyl; substituted phenyl; phenylalkyl or naphthylalkyl wherein the alkyl portion is $C_1$ - $C_8$ alkyl; $C_1$ - $C_8$ acyl or $C_3$ - $C_{10}$ cycloalkyl; or -$NR^8R^9$ may be taken together to form a ring having 4 - 10 ring atoms, which ring may be saturated or unsaturated, and may contain one or more hetero atoms in addition to the ring N, such as S, O or N within the ring; or optionally said ring having 4 - 10 ring atoms may be combined with a 2 - 4 membered carbon moiety to form a fused bicyclic ring, which may be saturated or unsaturated; or optionally said ring having 4 - 10 ring atoms may be combined with a 4 membered moiety containing at least two carbon atoms and up to two hetero atoms selected from S or O, to form a spirocycle ring system, which may be saturated or unsaturated; and wherein when -$NR^8R^9$ are taken together to form a ring, a fused ring system or a spirocycle ring system, said rings may be independently substituted with any of $C_1$ - $C_8$ alkyl; $C_1$ - $C_8$ alkoxy, phenyl, substituted phenyl (wherein phenyl may be substituted with any of the substituents listed hereinafter for $R^{10}$ or $R^{11}$ substituted phenyl), hydroxy, phenylalkyl or naphthylalkyl wherein the alkyl portion is $C_1$ - $C_8$, oxo or thioxo.
$R^{10}$ and $R^{11}$ are independently: $C_1$ - $C_5$ alkyl; $C_3$ - $C_7$ cycloalkyl; phenyl; phenyl substituted with one or more of $C_1$ - $C_8$ alkyl; $C_1$ - $C_8$ alkoxy, halogen, trifluoromethyl, $C_1$ - $C_8$ alkylthio, dialkylamino (wherein each alkyl is $C_1$ - $C_8$), $C_1$ - $C_8$ alkylamino, nitro or mono- or dialkylamino sulfonyl (wherein each alkyl is $C_1$ - $C_8$); $C_1$ - $C_5$ hydroxyalkyl; $C_1$ - $C_5$ alkoxy $C_1$ - $C_5$ alkyl; $C_1$ - $C_5$ alkoxy $C_1$ - $C_5$ alkylthio; an aromatic hydrocarbon group containing 1 or 2 hetero atoms selected from any of S, O or N; phenoxy; naphthoxy; phenylthio; naphthylthio; mono- or di-phenylamino; mono- or di-naphthylamino; hydroxyl; amino; amino- or mono- or di-$C_1$-$C_5$ alkylamino-carbonyl; nitro; cyano; halogen; trifluoromethyl; trifluoromethoxy; or amino- or mono- or di-$C_1$ - $C_5$ alkylamino-sulfonyl; and $R^{10}$ may also be oxo or thioxo, and
$R^{12}$ is: H; $C_1$-$C_8$ alkyl; hydroxyalkyl; or $C_1$-$C_8$ alkanoyl,
or an acceptable acid addition salt, hydrate, solvate, optical isomer or racemate thereof.

2. The compound of claim 1, wherein Ar is a fused ring system represented by the formula II as defined in claim 1.

3. The compound of claim 2, wherein D forms together with the two carbon atoms of the phenyl group an entirely or partly unsaturated ring consisting of 5 ring atoms, at least one of which is an oxygen atom;
wherein $R^{10}$ and $R^{11}$ are independently: $C_1$-$C_5$ alkyl; $C_1$-$C_5$ alkoxy; hydroxyl; nitro; cyano; halogen; or trifluoromethyl, with the proviso the $R^6$ is in the meta or ortho position in relation to the piperazine ring and wherein each of m and p has the value of 0 - 2.

**4.** The compound of claim 3, wherein m and p each equal 0.

**5.** The compound of claim 2, wherein when $R^{10}$ and $R^{11}$ comprise a $C_1$-$C_5$ alkyl group or a $C_3$-$C_7$ cycloalkyl group having not more than 10 carbon atoms including substituents.

**6.** The compound of claim 1, wherein Ar is phenyl substituted with a $C_1$-$C_8$ alkoxy group such as i-propoxy; A is N; n is 0; and $R^1$, $R^2$, $R^3$, and $R^4$ are H.

**7.** The compound of any one of claims 1 to 6, wherein $R^8$ and $R^9$ are taken together as $-NR^8R^9$ to form a ring having 4 - 8 ring atoms, which ring is saturated and contains up to one more hetero atom which is N, O or S, in addition to the N, and can optionally be substituted as defined in claim 1.

**8.** The compound of claim 7, wherein the 4 - 8 membered ring is unsubstituted.

**9.** The compound of claim 7, wherein the 4 - 8 membered ring is substituted, as defined in claim 1.

**10.** The compound of claim 1, wherein $R^8$ and $R^9$ are taken together as $-NR^8R^9$ to form a 4 - 10 membered ring and the ring is saturated prior to being combined with the 2 - 4 membered carbon moiety to form a fused ring.

**11.** The compound of claim 1, wherein a 4 membered moiety is used to form the spirocycle ring system and contains 2 oxygen atoms separated by 2 carbon atoms.

**12.** The compound of claim 6, wherein W is C or SO, wherein $R^5$ is O and wherein each of $R^6$ and $R^7$ are H.

**13.** The compound of claim 6, wherein W is C, wherein $R^5$ is S and wherein each of $R^6$ and $R^7$ are H.

**14.** The compound of claim 12, wherein $-NR^8R^9$ are taken together to form a saturated ring having 6 ring atoms.

**15.** The compound of claim 1, wherein Ar is substituted phenyl and it is substituted with one or more of: $C_1$ - $C_8$ alkyl; $C_1$ - $C_8$ alkoxy; cyano; $C_1$ - $C_8$ alkylthio; halogen; halo $C_1$-$C_5$ alkyl; trifluoromethyl; amino; or mono- or di-$C_1$-$C_5$ alkylamino.

**16.** The compound of claim 11, wherein Ar is substituted with one or more of $C_1$ - $C_8$ alkyl; $C_1$ - $C_8$ alkoxy; halogen; or halo $C_1$-$C_5$ alkyl and wherein $-NR^8R^9$ are taken together to form a saturated ring having 4 - 8 carbon ring atoms with the N being the only hetero atom in the ring.

**17.** A compound according to Claim 1, selected from:-

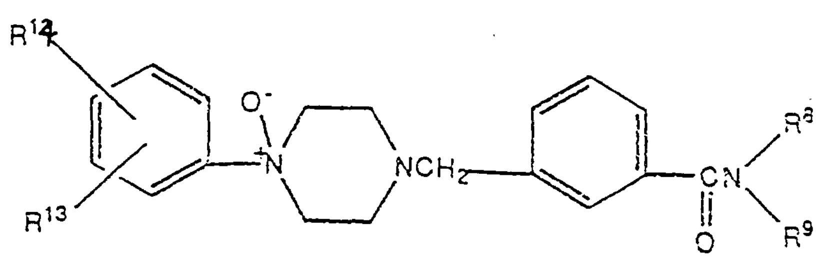

$I_2$

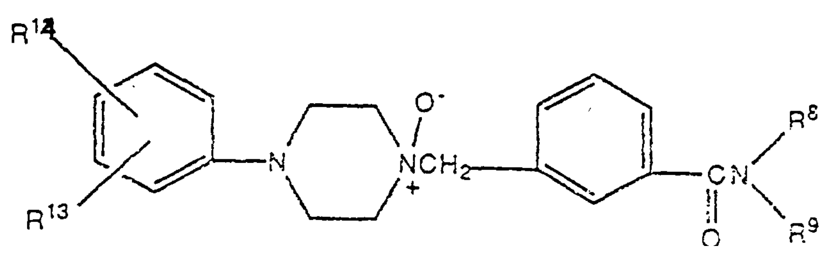

$I_3$

or

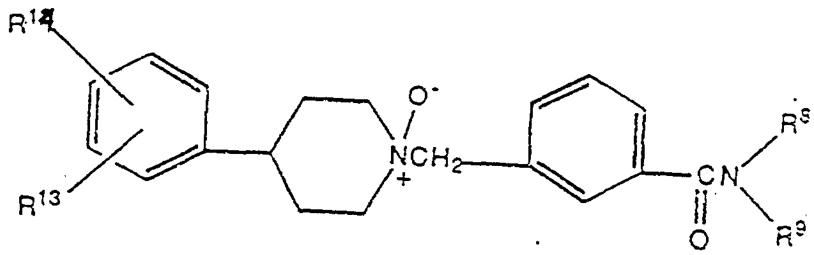

wherein $R^8$ and $R^9$ are as defined in Claim 1 and $R^{13}$ and $R^{14}$ are as defined as substituents for Ar in Claim 1, or a pharmaceutically acceptable acid addition salt, hydrate, solvate, isomer or racemate thereof.

**18.** The compound of claim 17, wherein $R^{12}$ is $C_1$ - $C_8$ alkoxy.

**19.** The compound of claim 18, wherein $-NR^8R^9$ are taken together to form a fully saturated ring containing 5 ring carbon atoms.

**20.** The compound of claim 19, wherein the $-NR^8R^9$ ring is independently substituted with any $C_1$ - $C_8$ alkyl.

**21.** The compound of claim 19, wherein the $-NR^8R^9$ ring is unsubstituted.

**22.** The compounds of Claim 1, which are 1-[3-[[4-[2(1-methylethoxy)phenyl]-4-oxide-1-piperazinyl]methyl]benzoyl] piperidine;
1-[3-[[4-[2-(1-methylethoxy)phenyl]-1-piperazinyl-1-oxide]methyl]benzoyl]piperidine; or
1-[3-[[4-[2-(1-methylethoxy)phenyl]-1-piperidinyl-1-oxide]methyl]benzoyl]-cis-2,6-dimenthylpiperidine.

**23.** A composition comprising the compound of any one of claims 1 to 22, and a pharmaceutically acceptable carrier, said compound being present in a therapeutically effective amount.

**24.** The compound of any one of claims 1 to 22 or the composition of claim 23 for use in treating a psychotic condition, such as schizophrenia, in an animal.

**Patentansprüche**

**1.** Verbindung, dargestellt durch die Formel I:

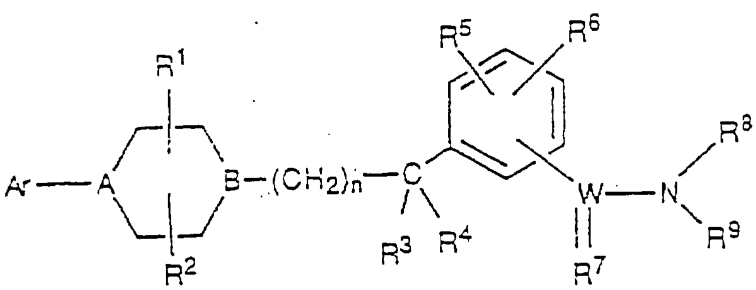

I

wobei

Ar ist: Phenyl oder Naphthyl, wahlweise substituiert mit einem oder mehreren $C_1$ — $C_8$-Alkyl, Cycloalkyl, $C_1$ — $C_8$-Alkoxy, Phenoxy, Naphthoxy, Hydroxyl, Trifluormethyl, Trifluormethoxy, Cyano, $C_1$ — $C_8$-Alkylthio, Halogen, Nitro, $C_1$ — $C_8$-Halogenalkylamino oder mono- oder di-Alkylamino, wobei jedes Alkyl $C_1$ — $C_8$ ist; Pyrimidinyl; Pyridinyl; Pyridazinyl; Pyrazinyl; Imidazyl; Pyrrol; Furan; Thiophen; Triazolyl; Thiazolyl; oder ein fusioniertes Ringsystem der Formel II:

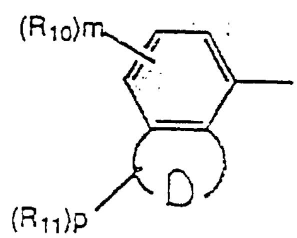

II

A ist: N; CH; oder $N^+$-$O^-$,
B ist: N; oder $N^+$-$O^-$;
unter der Bedingung, daß, wenn A N oder CH ist, B $N^+$-$O^-$ sein muß, und wenn A $N^+$-$O^-$ ist, B N sein muß,
D zusammen mit den zwei Kohlenstoffatomen der Phenylgruppe eine vollständig oder teilweise ungesättigte zyklische Gruppe bildet, die 5 — 7 Ringatome und innerhalb des Rings 0 — 3 Heteroatome von irgendeinem von O, S oder N hat, unter der Bedingung, daß die Summe der Anzahl an O- und S-Atomen höchstens 2 ist, wohingegen die N-Atome im Ring mit $R^{12}$ substituiert sein können,
W C oder SO ist,
$R^1$ und $R^2$ sind: H; oder $C_1$ — $C_4$-Alkyl,
m = 0 - 3,
n = 0 - 4,
p = 0 - 2,
$R^3$ und $R^4$ sind: entweder beide H; oder einer von ihnen ist H und der andere ist $C_1$ — $C_4$-Alkyl oder -Hydroxyl; oder beide sind als Sauerstoff zusammengenommen, um eine Carbonylgruppe zu bilden, unter der Bedingung, daß, wenn n = 0, $R^3$ und $R^4$ nicht als Sauerstoff zusammengenommen sein können,
$R^5$ und $R^6$ sind unabhängig voneinander: H; $C_1$ — $C_8$-Alkyl; $C_1$ — $C_8$-Alkoxy; Nitro; Halogen; Halogenalkyl; $C_1$ — $C_8$-Alkylthio; Amino; $C_1$ — $C_8$-mono- oder -di-Alkylamino; oder $C_1$ — $C_8$-Alkylamido,
$R^7$ ist: O oder S, wo W C ist; oder O, wo W SO ist,
$R^8$ und $R^9$ sind unabhängig voneinander: H; $C_1$ — $C_8$-Alkyl; $C_1$ — $C_8$-Aminoalkyl; Phenyl; substituiertes Phenyl; Phenylalkyl oder Naphthylalkyl, wobei der Alkylteil $C_1$ — $C_8$-Alkyl ist; $C_1$ — $C_8$-Acyl oder $C_3$ — $C_{10}$-Cycloalkyl; oder —$NR^8R^9$ kann zusammengenommen sein, um einen Ring zu bilden, der 4 — 10 Ringatome hat, wobei der Ring gesättigt oder ungesättigt sein kann und ein oder mehrere Heteroatome zusätzlich zu dem Ring-N enthalten kann, wie zum Beispiel S, O oder N innerhalb des Rings; oder wahlweise kann der Ring, der 4 — 10 Ringatome hat, mit einer 2 — 4-gegliederten Kohlenstoffeinheit verbunden sein, um einen fusionierten bizyklischen Ring zu bilden, welcher gesättigt oder ungesättigt sein kann; oder wahlweise kann dieser Ring, der 4 — 10 Ringatome hat, mit einer 4-gegliederten Einheit verbunden sein, die mindestens 2 Kohlenstoffatome und bis zu 2 Heteroatome enthält, ausgewählt aus S oder O, um ein spirozyklisches Ringsystem zu bilden, das gesättigt oder ungesättigt sein kann; und wobei, wenn —$NR^8R^9$ zusammengenommen sind, um einen Ring zu bilden, ein fusioniertes Ringsystem oder ein spirozyklisches Ringsystem entsteht, wobei die Ringe unabhängig voneinander mit irgendeinem von $C_1$ — $C_8$-Alkyl; $C_1$ — $C_8$-Alkoxy, Phenyl, substituiertes Phenyl (wobei Phenyl mit irgendeinem der im folgenden aufgeführten Substituenten für $R^{10}$ - oder $R^{11}$-substituiertes Phenyl substituiert sein kann), Hydroxy, Phenylalkyl oder Naphthylalkyl, wobei der Alkylanteil $C_1$ — $C_8$ ist, Oxo oder Thioxo, substituiert sein kann.
$R^{10}$ und $R^{11}$ sind unabhängig voneinander: $C_1$ — $C_5$-Alkyl; $C_3$ — $C_7$-Cycloalkyl; Phenyl; Phenyl, substituiert mit ein oder mehrere aus $C_1$ — $C_8$-Alkyl; $C_1$ — $C_8$-Alkoxy, Halogen, Trifluormethyl, $C_1$ — $C_8$-Alkylthio, Dialkylamino (wobei jedes Alkyl $C_1$ — $C_8$ ist), $C_1$ — $C_8$-Alkylamino, Nitro oder Mono- oder Dialkylaminosulfonyl (wobei jedes Alkyl $C_1$ — $C_8$ ist); $C_1$ — $C_5$-Hydroxyalkyl; $C_1$ — $C_5$-Alkoxy-$C_1$ — $C_5$-Alkyl; $C_1$ — $C_5$-Alkoxy-$C_1$ — $C_5$-Alkylthio; eine aromatische Kohlenwasserstoffgruppe, die 1 oder 2 Heteroatome enthält, ausgewählt aus irgendeinem von S, O oder N; Phenoxy; Naphthoxy; Phenylthio; Naphthylthio; mono- oder di-Phenylamino; mono- oder di-Naphthylamino; Hydroxyl; Amino; amino- oder mono- oder di-$C_1$ — $C_5$-Alkylamino-Carbonyl; Nitro; Cyano; Halogen; Trifluormethyl; Trifluormethoxy; oder amino- oder mono- oder di-$C_1$ — $C_5$-Alkylamino-Sulfonyl; und $R^{10}$ kann auch

Oxo oder Thioxo sein, und
$R^{12}$ ist: H; $C_1$ — $C_8$-Alkyl; Hydroxyalkyl; oder $C_1$ — $C_8$-Alkanoyl,
oder ein akzeptables Salz nach Säurezugabe, Hydrat, Solvat, optisches Isomer oder Racemat desselben.

**2.** Verbindung nach Anspruch 1, wobei Ar ein fusioniertes Ringsystem ist, dargestellt durch die Formel II, wie in Anspruch 1 definiert.

**3.** Verbindung nach Anspruch 2, wobei D zusammen mit den 2 Kohlenstoffatomen der Phenylgruppe einen vollständig oder teilweise ungesättigten Ring bildet, der aus 5 Ringatomen besteht, von denen mindestens eins ein Sauerstoffatom ist;
wobei $R^{10}$ und $R^{11}$ unabhängig voneinander: $C_1$ — $C_5$-Alkyl; $C_1$ — $C_5$-Alkoxy; Hydroxyl; Nitro; Cyano; Halogen; oder Trifluormethyl sind, unter der Bedingung, daß $R^6$ in Bezug auf den Piperazinring in der meta- oder ortho-Stellung ist und wobei jedes von m und p den Wert 0 — 2 hat.

**4.** Verbindung nach Anspruch 3, wobei m und p jeweils gleich 0 sind.

**5.** Verbindung nach Anspruch 2, wobei diese, wenn $R^{10}$ und $R^{11}$ eine $C_1$ — $C_5$-Alkylgruppe oder eine $C_3$ — $C_7$-Cycloalkylgruppe umfassen, nicht mehr als 10 Kohlenstoffatome, einschließlich der Substituenten, hat.

**6.** Verbindung nach Anspruch 1, wobei Ar Phenyl, substituiert mit einer $C_1$ — $C_8$-Alkoxygruppe wie zum Beispiel i-Propoxy, ist; A N ist; n O ist; und $R^1$, $R^2$, $R^3$ und $R^4$ H sind.

**7.** Verbindung nach einem der Ansprüche 1 bis 6, wobei $R^8$ und $R^9$ als —$NR^8R^9$ zusammengenommen werden, um einen Ring zu bilden, der 4 — 8 Ringatome hat, wobei der Ring gesättigt ist und zusätzlich zu dem N bis zu einem Heteroatom mehr enthalten kann, welches N, O oder S ist und wahlweise, wie in Anspruch 1 definiert, substituiert sein kann.

**8.** Verbindung nach Anspruch 7, wobei der 4 — 8-gegliederte Ring nicht-substituiert ist.

**9.** Verbindung nach Anspruch 7, wobei der 4 — 8-gegliederte Ring substituiert ist, wie in Anspruch 1 definiert.

**10.** Verbindung nach Anspruch 1, wobei $R^8$ und $R^9$ als —$NR^8R^9$ zusammengenommen werden, um eine 4 — 10-gegliederten Ring zu bilden und der Ring gesättigt ist, bevor er mit der 2 — 4-gegliederten Kohlenstoffeinheit verbunden wird, um einen fusionierten Ring zu bilden.

**11.** Verbindung nach Anspruch 1, wobei eine 4-gegliederte Einheit verwendet wird, um das spirozyklische Ringsystem zu bilden und 2 Sauerstoffatome, getrennt durch 2 Kohlenstoffatome, enthält.

**12.** Verbindung nach Anspruch 6, wobei W C oder SO ist, wobei $R^5$ O ist und wobei jeder von $R^6$ und $R^7$ H ist.

**13.** Verbindung nach Anspruch 6, wobei W C ist, wobei $R^5$ S ist und wobei jeder von $R^6$ und $R^7$ H ist.

**14.** Verbindung nach Anspruch 12, wobei —$NR^8R^9$ zusammengenommen werden, um einen gesättigten Ring zu bilden, der 6 Ringatome hat.

**15.** Verbindung nach Anspruch 1, wobei Ar substituiertes Phenyl ist und dieses mit ein oder mehreren von: $C_1$ — $C_8$-Alkyl; $C_1$ — $C_8$-Alkoxy; Cyano; $C_1$ — $C_8$-Alkylthio; Halogen; Halogen-$C_1$ — $C_5$-alkyl; Trifluormethyl; Amino; oder mono- oder di-$C_1$ — $C_8$-Alkylamino, substituiert ist.

**16.** Verbindung nach Anspruch 11, wobei Ar mit einem oder mehreren von $C_1$ — $C_8$-Alkyl; $C_1$ — $C_8$-Alkoxy; Halogen; oder Halogen-$C_1$ — $C_5$-alkyl substituiert ist und wobei —$NR^8R^9$ zusammengenommen sind, um einen gesättigten Ring zu bilden, der 4 — 8 Kohlenstoffringatome hat, indem das N das einzige Heteroatom im Ring ist.

**17.** Verbindung nach Anspruch 1, ausgewählt aus:

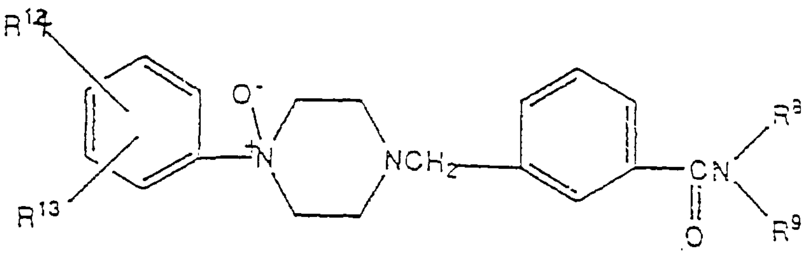

Ia

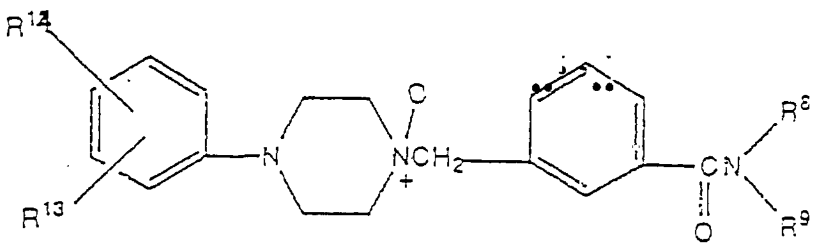

Ib

oder

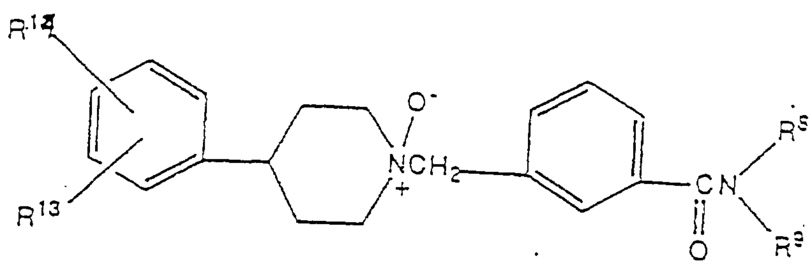

Ic

wobei $R^8$ und $R^9$ wie in Anspruch 1 definiert sind und $R^{13}$ und $R^{14}$ wie die für Ar in Anspruch 1 definierten Substituenten oder ein pharmazeutisch akzeptables Salz nach Säurezugabe, Hydrat, Solvat, Isomer oder Racemat derselben sind.

**18.** Verbindung nach Anspruch 17, wobei $R^{12}$ $C_1$ — $C_8$-Alkoxy ist.

**19.** Verbindung nach Anspruch 18, wobei —$NR^8R^9$ zusammengenommen sind, um einen vollständig gesättigten Ring zu bilden, der 5 Ringkohlenstoffatome enthält.

**20.** Verbindung nach Anspruch 19, wobei der —$NR^8R^9$-Ring unabhängig mit irgendeinem $C_1$ - $C_8$-Alkyl substituiert ist.

**21.** Verbindung nach Anspruch 19, wobei der —$NR^8R^9$-Ring nicht-substituiert ist.

**22.** Verbindungen nach Anspruch 1, die 1-[3-[[4-[2-(1-Methylethoxy)phenyl]-4-oxid-1-piperazinyl]methyl]benzoyl]piperidin;
1-[3-[[4-[2-(1-Methylethoxy)phenyl]-1-piperazinyl-1-oxid]methyl]benzoyl]piperidin; oder
1-[3-[[4-[2-(1-Methylethoxy)phenyl]-1-piperidinyl-1-oxid]methyl]benzoyl]-cis-2,6-dimethylpiperidin sind.

**23.** Zusammensetzung, die die Verbindung nach einem der Ansprüche 1 bis 22 und einen pharmazeutisch akzeptablen Träger umfaßt und die Verbindung in einer therapeutisch wirksamen Menge vorhanden ist.

**24.** Verbindung nach einem der Ansprüche 1 bis 22 oder die Zusammensetzung nach Anspruch 23 zur Verwendung

bei der Behandlung eines psychotischen Zustandes, wie zum Beispiel Schizophrenie, bei einem Tier.

## Revendications

**1.** Composé représenté par la formule I:

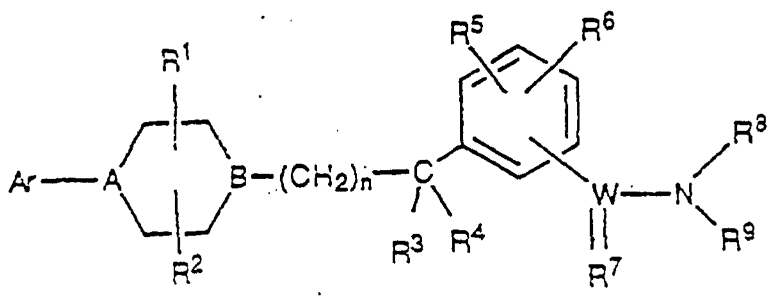

I

où:

Ar est phényle ou naphtyle, facultativement substitué par un ou plusieurs d'alkyle $C_1$-$C_8$, cycloalkyle, alcoxy $C_1$-$C_8$, phénoxy, naphtoxy, hydroxyle, trifluorométhyle, trifluorométhoxy, cyano, alkylthio $C_1$-$C_8$, halogène, nitro, haloalkyl amino $C_1$-$C_8$ ou mono- ou dialkylamino où chaque alkyle est $C_1$-$C_8$; pyrimidinyle; pyridinyle; pyridazinyle; pyrazinyle; imidazyle, pyrrole; furane; thiophène; triazolyle; thiazolyle; ou un système d'un cycle fusionné de la formule II:

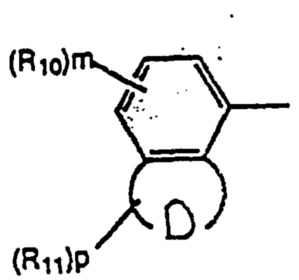

II

A est : N; CH; ou $N^+$-$O^-$,
B est: N; ou $N^+$-$O^-$;
à condition que quand A est N ou CH, B doive être $N^+$-$O^-$ et quand A est $N^+$-$O^-$ B doit être N,
D, avec les deux atomes de carbone du groupe phényle, forme un groupe cyclique entièrement ou partiellement insaturé ayant 5 - 7 atomes dans le cycle et dans le cycle 0 - 3 hétéroatomes à partir de l'un de O, S ou N, à condition que la somme du nombre des atomes de O et S soit au plus de 2, tandis que les atomes de N dans le cycle peuvent être substitués par $R^{12}$,
W est C ou SO,
$R^1$ et $R^2$ sont : H; ou alkyle $C_1$ - $C_4$,
m = 0 - 3,
n = 0 -4,
p = 0 - 2,
$R^3$ et $R^4$ sont: soit tous deux H; ou bien l'un d'entre eux est H et l'autre est alkyle $C_1$ - $C_4$ ou hydroxyle; ou bien tous deux sont pris ensemble en tant qu'oxygène pour constituer un groupe carbonyle, à condition que quand n = 0, $R^3$ et $R^4$ ne puissent être pris ensemble comme oxygène,
$R^5$ et $R^6$ sont indépendamment : H; alkyle $C_1$-$C_8$; alcoxy $C_1$-$C_8$; nitro; halogène; haloalkyle; alkylthio $C_1$-$C_8$; amino; mono ou dialkylamino $C_1$ - $C_8$ ou alkylamido $C_1$ - $C_8$,

$R^7$ est: O ou S si W est C; ou bien O si W est SO,

$R^8$ et $R^9$ sont indépendamment : H; alkyle $C_1$-$C_8$; aminoalkyle $C_1$-$C_8$; phényle; phényle substitué; phénylalkyle ou naphtylalkyle où la portion d'alkyle est alkyle $C_1$-$C_8$; acyle $C_1$-$C_8$ ou cycloalkyle $C_3$-$C_{10}$; ou bien -$NR_8R_9$ peuvent être pris ensemble pour former un cycle ayant 4 - 10 atomes dans le cycle, lequel cycle peut être saturé ou insaturé et peut contenir un ou plusieurs hétéroatomes en plus de N du cycle, comme S, O ou N dans le cycle; ou bien facultativement ledit cycle ayant 4 - 10 atomes dans le cycle peut être combiné à une fraction de carbone à 2 - 4 membres pour former un cycle bicyclique fusionné, qui peut être saturé ou insaturé; ou bien facultativement ledit cycle ayant 4 - 10 atomes dans le cycle peut être combiné à une fraction à 4 membres contenant au moins deux atomes de carbone et jusqu'à deux hétéroatomes sélectionnés parmi S ou O, pour former un cycle spirocyclique, qui peut être saturé ou insaturé, et où quand -$NR^8R^9$ sont pris ensemble pour former un cycle, un système d'un cycle fusionné ou un système d'un cycle spirocyclique, lesdits cycles peuvent être indépendamment substitués par l'un de alkyle $C_1$ - $C_8$; alcoxy $C_1$ - $C_8$, phényle, phényle substitué (où phényle peut être substitué par l'un des substituants dont la liste est donnée ci-après pour phényle substitué par $R^{10}$ ou $R^{11}$), hydroxy, phénylalkyle ou naphtylalkyle où la portion d'alkyle est $C_1$ - $C_8$, oxo ou thioxo.

$R^{10}$ et $R^{11}$ sont indépendamment : alkyle $C_1$ - $C_5$; cycloalkyle $C_3$ - $C_7$; phényle; phényle substitué par un ou plusieurs de alkyle $C_1$-$C_8$; alcoxy $C_1$-$C_8$, halogène, trifluorométhyle, alkylthio $C_1$-$C_8$, dialkylamino (où chaque alkyle est $C_1$ - $C_8$, alkylamino $C_1$ - $C_8$, nitro ou mono- ou dialkylaminosulfonyle (où chaque alkyle est $C_1$ - $C_8$); hydroxyalkyle $C_1$-$C_5$; alcoxy $C_1$-$C_5$ alkyle $C_1$-$C_5$; alcoxy $C_1$-$C_5$ alkylthio $C_1$-$C_5$; un groupe hydrocarbure aromatique contenant 1 ou 2 hétéroatomes sélectionnés parmi l'un de S, O ou N; phénoxy; naphtoxy; phénylthio; naphtylthio; mono- ou di-phénylamino; mono- ou di-naphtylamino; hydroxyle; amino; amino- ou mono- ou di-alkylaminocarbonyle $C_1$-$C_5$; nitro; cyano; halogène; trifluorométhyle; trifluorométhoxy; ou amino- ou mono- ou di-alkylaminosulfonyle $C_1$-$C_5$; et $R^{10}$ peut également être oxo ou thioxo, et

$R^{12}$ est H; alkyle $C_1$-$C_8$; hydroxyalkyle; ou alcanoyle $C_1$-$C_8$, ou son sel d'addition d'acide, hydrate, solvate, isomère optique ou racémate acceptable.

2. Composé de la revendication 1, où Ar est un système d'un cycle fusionné représenté par la formule II telle que définie à la revendication 1.

3. Composé de la revendication 2, où D forme, avec les deux atomes de carbone du groupe phényle, un cycle entièrement ou partiellement insaturé consistant en 5 atomes dans le cycle, dont au moins un est un atome d'oxygène;
où $R^{10}$ et $R^{11}$ sont indépendamment: alkyle $C_1$-$C_5$; alcoxy $C_1$-$C_5$; hydroxyle; nitro; cyano; halogène; ou trifluorométhyle, à condition que $R^6$ soit à la position méta ou ortho relativement au cycle de pipérazine et où chacun de m et p a la valeur de 0 - 2.

4. Composé de la revendication 3, où m et p sont chacun égal à 0.

5. Composé de la revendication 2, où quand $R^{10}$ et $R^{11}$ comprennent un groupe alkyle $C_1$-$C_5$ ou un groupe cycloalkyle $C_3$-$C_7$ n'ayant pas plus de 10 atomes de carbone comprenant des substituants.

6. Composé de la revendication 1, où Ar est phényle substitué par un groupe alcoxy $C_1$-$C_8$, tel que i-propoxy; A est N; n est 0; et $R^1$, $R^2$, $R^3$, et $R^4$ sont H.

7. Composé de l'une quelconque des revendications 1 à 6, où $R^8$ et $R^9$ sont pris ensemble en tant que -$NR^8R^9$ pour former un cycle ayant 4 - 8 atomes dans le cycle, lequel cycle est saturé et contient jusqu'à un hétéroatome de plus qui est N, O ou S, en addition à N, et peut facultativement être substitué comme défini à la revendication 1.

8. Composé de la revendication 7, où le cycle à 4 - 8 membres est non substitué.

9. Composé de la revendication 7, où le cycle à 4 - 8 membres est substitué, comme défini à la revendication 1.

10. Composé de la revendication 1, où $R^8$ et $R^9$ sont pris ensemble en tant que -$NR^8R^9$ pour former un cycle à 4 - 10 membres et le cycle est saturé avant d'être combiné à la fraction de carbone à 2 - 4 membres pour former un cycle fusionné.

11. Composé de la revendication 1, où une fraction à 4 membres est utilisée pour former le système du cycle spirocyclique et contient 2 atomes d'oxygène séparés par deux atomes de carbone.

**12.** Composé de la revendication 6, où W est C ou SO, où $R^5$ est O et où chacun de $R^6$ et $R^7$ sont H.

**13.** Composé de la revendication 6, où W est C, où $R^5$ est S et où chacun de $R^6$ et $R^7$ est H.

**14.** Composé de la revendication 12, où $-NR^8R^9$ sont pris ensemble pour former un cycle saturé ayant 6 atomes dans le cycle.

**15.** Composé de la revendication 1, où Ar est phényle substitué et il est substitué par un ou plusieurs de: alkyle $C_1$ - $C_8$; alcoxy $C_1$ - $C_8$; cyano; alkylthio $C_1$ - $C_8$; halogène; haloalkyle $C_1$ - $C_5$; trifluorométhyle; amino; ou mono- ou di-alkylamino $C_1$-$C_5$.

**16.** Composé de la revendication 11, où Ar est substitué par un ou plusieurs d'alkyle $C_1$ - $C_8$; alcoxy $C_1$-$C_8$; halogène; ou haloalkyle $C_1$-$C_5$ et où $-NR_8R_9$ sont pris ensemble pour former un cycle saturé ayant 4 - 8 atomes de carbone dans le cycle avec le N qui est le seul hétéroatome dans le cycle.

**17.** Composé selon la revendication 1, sélectionné parmi:

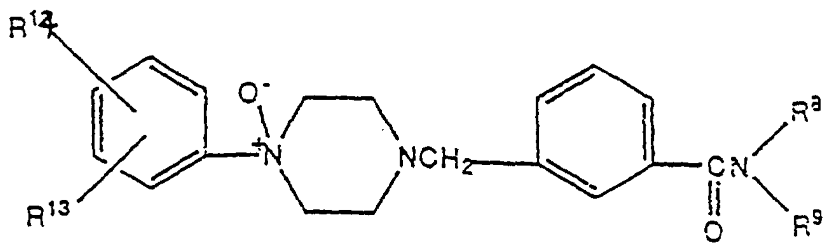

Ia

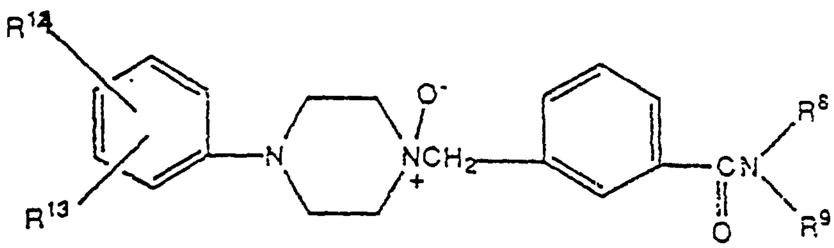

Ib

or

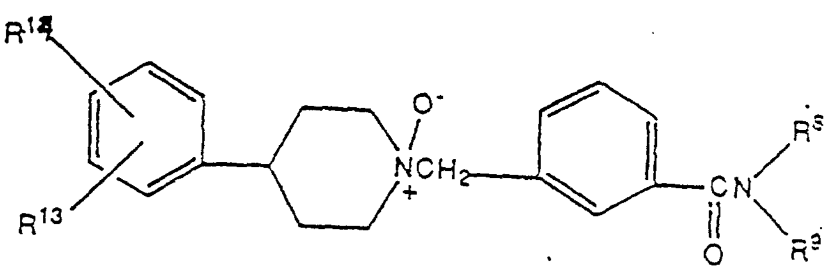

Ic

où $R^8$ et $R^9$ sont tels que définis à la revendication 1 et $R^{13}$ et $R^{14}$ sont tels que définis comme substituants pour Ar à la revendication 1, ou bien son sel d'addition d'acide, hydrate, solvate, isomère ou racémate pharmaceutiquement acceptable.

**18.** Composé de la revendication 17, où $R^{12}$ est alcoxy $C_1$ - $C_8$.

**19.** Composé de la revendication 18, où $-NR^8R^9$ sont pris ensemble pour former un cycle complètement saturé contenant 5 atomes de carbone dans le cycle.

**20.** Composé de la revendication 19, où le cycle $-NR^8R^9$ est indépendamment substitué par tout alkyle $C_1$ - $C_8$.

**21.** Composé de la revendication 19 où le cycle $-NR^8R^9$ est non substitué.

**22.** Composés de la revendication 1, qui sont 1-[3-[[4-[2(1-méthyléthoxy)phényl]-4-oxyde-1-pipérazinyl]méthyl]-benzoyl]pipéridine;
1-[3-[[4-[2(1-méthyléthoxy)phényl]-4-oxyde-1-pipérazinyl-1-oxyde]méthyl]benzoyl]pipéridine; ou
1-[3-[[4-[2(1-méthyléthoxy)phényl]-1-pipéridinyl-1-oxyde]-méthyl]benzoyl]-cis-2,6-dimenthylpipéridine.

**23.** Composition comprenant le composé de l'une quelconque des revendications 1 à 22, et un support pharmaceutiquement acceptable, ledit composé étant présent en une quantité thérapeutiquement efficace.

**24.** Composé de l'une quelconque des revendications 1 à 22 ou la composition de la revendication 23 pour une utilisation dans le traitement d'une condition psychotique, comme la schizophrénie, chez un animal.